# EUROPEAN PATENT APPLICATION

(11) **EP 1 088 535 A1**
(43) Date of publication of application: **04.04.2001**
(21) Application number: 99922612.9
(22) Date of filing: 01.06.1999
(51) Int. Cl.: A61F 13/00, A61K 9/70, A61K 31/215, A61K 35/78

(54) **SILK CLOTHES FOR PROTECTING AFFECTED PARTS**

(30) Priority: 03.06.1998 JP 15418898; 17.02.1999 JP 3911299
(71) Applicant: Tsuchida, Yuzo, Shinagawa-ku, Tokyo 142-0062 (JP)
(72) Inventor: Tsuchida, Yuzo, Shinagawa-ku, Tokyo 142-0062 (JP)
(74) Representative: Altenburg, Udo, Dipl.-Phys.
(86) International application number: JP9902905
(87) International publication number: WO9962444

(57) **Abstract**

Silk clothes for protecting affected parts (incised wound, burn, tumor, bedsore, etc.) which also contribute to the prevention of suppurating. These clothes are composed of a knitted woven silk fabric or non-woven silk fabric (silk floss, etc.) containing either *Sasa veitchii* or a parabenzoic acid ester or both of the same and being adhered to a non-woven fabric made of cellulose, polyester or polyurethane, etc. or a polyurethane-reinforced non-woven fabric made of cellulose, etc.

## Description

### Technical Field

The present invention relates to protective silk cloths, adapted to be applied to affected parts, such as cuts, burns, tumors, bedsores, and the like, to protect such affected parts.

### Background Technology

Protecting affected parts with protective silk cloths is known to the public by means of Utility Model Registration Nos. 3,040,123 and 3,040,661 that were devised by the inventor of the present patent application.

Conventional protective silk cloths of prior art would not cause a patient to feel indisposed with use thereof, because of excellent conformability of silk itself to the skin, and because of virulence absorbability of silk absorbing things detrimental to cure, such as humidity, suppuration, dirt, and the like, thereby contributing to improvement of curing effects for affected parts. Because of lacking antibacterialness, however, such protective silk cloths were likely to cause suppuration in the affected parts, such as cuts, burns, bedsores, and the like, unless treatment, such as applying antibacterial agent and the like, was provided to the affected parts separately.

The present invention is made to solve the problem described above. Thus, an object of the present invention is to provide a protective silk cloth having antibacterialness.

### Disclosure of The Invention

In order to achieve the object described above, means employed by the present invention is to provide a protective cloth for affected parts, comprising a piece of silk fabric made to contain extract of *Sasa veitchii* (Carriere) Rehder (common name: Kuma Zasa) (hereinafter referred to as "Sasa veitchii") that has antibacterialness, and/or an antibacterial agent, such as a parabenzoic ester, and the like.

The silk fabric may be knitted-or-woven fabric made by knitting or weaving raw silk or spun silk yarn, or non-woven fabric made by twisting floss silk, or the like, into fabric-like texture. The protective silk cloth for affected parts may also comprise a piece of this silk fabric, such as floss silk, or the like, adhered by means of adhesive, to a piece of non-woven fabric made of either of cellulose (produced from wooden pulp, or the like), polyester, polyurethane, or the like, or to a piece of non-woven fabric made of cellulose, reinforced with polyurethane or the like.

For parabenzoic ester, a solution may be used of either n-butyl-p-hydroxybenzoic acid or n-propyl-p-hydroxybenzoic acid, both well known as an antibacterial agent, solved into alcohol, such as ethanol.

For Sasa veitchii extract, a Sasa veitchii extract may be used that used to be applied, as an agent having antibacterialness, to affected parts, such as burns, and the like.

### Brief Description of The Drawings

FIG. 1 is a perspective view of a protective cloth, according to the present invention; and
FIG. 2 is a perspective view of a protective cloth adhered to a piece of non-woven fabric, according to the present invention.

### Best Embodiments of The Invention

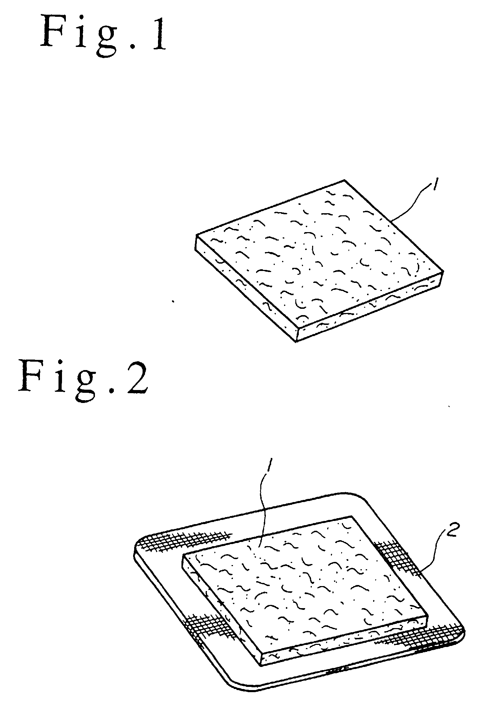

As shown in FIG. 1. a protective cloth, according to the present invention, may be manufactured by spraying with Sasa veitchii extract, and/or an alcoholic solution of parabenzoic ester, and then drying, a piece of silk fabric 1 of floss silk, or the like, or by impregnating with Sasa veitchii extract and/or an alcoholic solution of parabenzoic ester, and then drying, a piece of silk fabric of floss silk, or the like. Also, as shown in FIG. 2, if required, a protective cloth, according to the present invention, may be manufactured by adhering such piece of silk fabric 1, to a piece of non-woven fabric 2 made of either cellulose (produced from wooden pulp, or the like), polyester, or polyurethane, or to a piece of non-woven fabric 2 made of cellulose reinforced with polyurethane, or the like.

In addition to the conformability to the skin and the absorbability, of silk itself, a protective cloth manufactured, as described above, contains one or more from Sasa veitchii extract, that used to be applied, as an agent having antibacterialness, from the old times, and parabenzoic esters, well known as antibacterial agents, and is therefore equipped with antibacterialness, thereby useful for preventing the affected parts from suppurating.

These protective cloths, mainly made of such natural materials, and protective cloths, made of synthetic materials not including vinyl chloride, are inexpensive and, furthermore, are capable of being disposed as waste without causing environmental pollution.

As described above, in addition to the conformability to the skin and the virulence absorbability for absorbing humidity, suppuration, and the like, of silk itself, a protective silk cloth, according to the present invention, has antibacterialness, especially strong antibacterialness against Pseudomonas aeruginosa, provided by agents, such as Sasa veitchii extract and parabenzoic esters, contained therein. Thus, excellent effects are obtained in that the protective silk cloths, according to the present invention, are not only effective against affected parts, such as tumors, having inflammation, but are also useful for preventing the affected parts, such as cuts, burns, bedsores, and the like, from suppurating.

Next, the present invention will be discussed based on antibacterialness tests.

In the tests, sample floss silk is cut out to obtain circular disks of about a 2-cm diameter, which are then put in respective Petri dishes separately, as specimens. After test bacteria solution of 0.1 ml (milliliter) is dripped on the testing surface of each specimen, each dish has its cover secured and is preserved for 19 hours under the conditions of 35 degrees C (deg.C) temperature and 90% relative humidity. After the preservation, each specimen is washed out using sterile, physiological salt solution of 1.9 ml, to obtain a recovery solution. With the recovery solution, the number of live bacteria is measured by means of the agar plating method (cultivation for 24 hours at 35 deg.C) using normal agar. In case no bacteria are detected, the number of bacteria after preservation is denoted as "<50."

Test bacteria solution described herein is obtained by transplanting test bacteria to an SCD cultivation medium for subsequent precultivation for nine hours at 35 deg.C, and thereafter diluting the precultivation solution with sterile, physiological salt solution so as to obtain the number of bacteria equal to 10⁵/ml.

### [Illustrative Embodiment 1 (Escherichia coli)]

As shown in Table 1, floss silk was soaked in five different types of test solutions, respectively, and dried after the soaking, to obtain Specimens No.1 - No.5. Untreated floss silk was denoted as Specimen No.6.

**(Table 1)**

| Specimen | Test Solution |
|---|---|
| No.1 | Sasa veitchii extract; |
| No.2 | Ethanol containing n-butyl-p-hydroxybenzoic acid by 10% (also referred to as 10%-butyl-parabenzoic-ester ethanol); |
| No.3 | Ethanol containing n-butyl-p-hydroxybenzoic acid by 20% (also referred to as 20%-butyl-parabenzoic-ester ethanol); |
| No.4 | Ethanol containing n-propyl-p-hydroxybenzoic acid by 10% (also referred to as 10%-propyl-parabenzoic-ester ethanol); |
| No.5 | Ethanol containing n-propyl-p-hydroxybenzoic acid by 20% (also referred to as 20%-propyl-parabenzoic-ester ethanol); and |
| No.6 | Untreated. |

Antibacterialness tests were applied to each specimen by means of Escherichia coli (Escherichia coli IFO 3972) to obtain a result as shown in Table 2.

**(Table 2)**

| A Result of Measuring the Number of Live Bacteria | | |
|---|---|---|
| Specimen (Test Solution) | A | B |
| No.1 (Sasa veitchii extract) | 4.60x10⁵ | <50 |
| No.2 (10%-butyl-parabenzoic-ester ethanol) | do. | 8.0x10⁵ |
| No.3 (20%-butyl-parabenzoic-ester ethanol) | do. | <50 |
| No.4 (10%-propyl-parabenzoic-ester ethanol) | do. | <50 |
| No.5 (20%-propyl-parabenzoic-ester ethanol) | do. | <50 |
| No.6 (Untreated) | do. | 3.30x10⁶ |
| (where A and B denote the number of inocula bacteria and the number of bacteria after preservation, respectively.) | | |

From Table 2, it was confirmed that Specimens Nos.1, 3, 4 and 5, containing Sasa veitchii extract, 20%-butyl-parabenzoic-ester ethanol, and 10%- and 20%-propyl-parabenzoic-ester ethanol, respectively, exhibited sufficient antibacterialness against Escherichia coli. However, both Specimen No.2 that contained 10%-butyl-parabenzoic-ester ethanol, and Specimen No.6 that was untreated, did not show sufficient antibacterialness against Escherichia coli.

### [Illustrative Embodiment 2 (Pseudomonas aeruginos)]

As shown in Table 3. Specimen No.7 was obtained by having floss silk soaked in a 1-in-6 diluted Sasa veitchii extract, and dried after the soaking, with Specimen No.8 obtained by having Specimen No.7 further gas sterilized, while Specimen No.9 was obtained by having floss silk soaked in a mixture solution in equivalent ratios of a 1%-butyl-parabenzoic-ester-containing ethanol and a 1-in-10 diluted Sasa veitchii extract, and dried after the soaking. Untreated floss silk was denoted as Specimen No.10.

**(Table 3)**

| Specimen | Test Solution |
|---|---|
| No.7 | 1-in-6 diluted Sasa veitchii extract; |
| No.8 | 1-in-6 diluted Sasa veitchii extract, gas sterilized; |
| No.9 | Mixture solution; and |
| No.10 | Untreated. |

Antibacterialness tests were applied to these specimens by means of Pseudomonas aeruginos (Pseudomonas aeruginos IFO 13275) to obtain a result as shown in Table 4.

**(Table 4)**

| Specimen (Test Solution) | A | B |
|---|---|---|
| No.7 (1-in-6 diluted Sasa veitchii extract) | 8.10x10⁵ | <50 |
| No.8 (No.7 Test Solution, further gas sterilized) | do. | <50 |
| No.9 (Mixture solution) | do. | <50 |
| No.10 (Untreated) | do. | 1.90x10⁷ |
| (where A and B denote the number of inocula bacteria and the number of bacteria after preservation, respectively.) | | |

Table 4 confirms that Specimens Nos.7 and 8, containing the 1-in-6 diluted Sasa veitchii extract, and Specimen No.9, containing the mixture solution of the 1-in-10 diluted Sasa veitchii extract and the 1%-butyl-parabenzoic-ester-containing ethanol, respectively, exhibited sufficient antibacterialness against Pseudomonas aeruginos, where no significant differences were found in the antibacterialness between Specimen No.7, which was made to contain the 1-in-6 diluted Sasa veitchii extract and just dried thereafter, and Specimen No.8, which was obtained by further gas sterilizing such Specimen No.7. However, Specimen No.10 that was untreated, did not show antibacterialness against Pseudomonas aeruginos.

### [Illustrative Embodiment 3 (Staphylococcus aureus)]

As shown in Table 5, Specimen No.11 was obtained by having floss silk soaked in a 1-in-6 diluted Sasa veitchii extract, and dried after the soaking, with Specimen No.12 obtained by having Specimen No.11 further gas sterilized, while Specimen No.13 was obtained by having floss silk soaked in a mixture solution in equivalent ratios of a 1%-butyl-parabenzoic-ester-containing ethanol and a 1-in-10 diluted Sasa veitchii extract, and dried after the soaking. Untreated floss silk was denoted as Specimen No.14.

**(Table 5)**

| Specimen | Test Solution |
|---|---|
| No.11 | 1-in-6 diluted Sasa veitchii extract; |
| No.12 | 1-in-6 diluted Sasa veitchii extract, gas sterilized; |
| No.13 | Mixture solution; and |
| No.14 | Untreated. |

Antibacterialness tests were applied to these specimens by means of Staphylococcus aureus (Staphylococcus aureus IFO 12732) to obtain a result as shown in Table 6.

**(Table 6)**

| Specimen (Test Solution) | A | B |
|---|---|---|
| No.11 (1-in-6 diluted Sasa veitchii extract) | 4.00x10⁵ | <50 |
| No.12 (No.7 Test Solution)(further gas sterilized) | do. | 9.6x10³ |
| No.13 (Mixture solution) | do. | <50 |
| No.14 (Untreated) | do. | 8.70x10⁵ |
| (where A and B denote the number of inocula bacteria and the number of bacteria after preservation, respectively.) | | |

From Table 6, it was confirmed that Specimen No.11, containing the 1-in-6 diluted Sasa veitchii extract, and Specimen No.13, containing the mixture solution of the 1-in-10 diluted Sasa veitchii extract and the 1%-butyl-parabenzoic-ester-containing ethanol, respectively, exhibited strong antibacterialness against Staphylococcus aureus, while Specimen No.12 that was obtained by gas sterilizing floss silk containing the 1-in-10 diluted Sasa veitchii extract, was inferior in antibacterialness against Staphylococcus aureus than Specimen No.11 that was not gas sterilized. Specimen No.14 that was untreated, did not show antibacterialness against Staphylococcus aureus.

### [Illustrative Embodiment 4 (Escherichia coli)]

As shown in Table 7, Specimen No.15 was obtained by having floss silk soaked in a 1-in-6 diluted Sasa veitchii extract, and dried after the soaking, with Specimen No.16 obtained by having Specimen No.15 further gas sterilized, while Specimen No.17 was obtained by having floss silk soaked in a mixture solution in equivalent ratios of a 1%-butyl-parabenzoic-ester-containing ethanol and a 1-in-10 diluted Sasa veitchii extract, and dried after the soaking. Untreated floss silk was denoted as Specimen No.18.

**(Table 7)**

| Specimen | Test Solution |
|---|---|
| No.15 | 1-in-6 diluted Sasa veitchii extract; |
| No.16 | 1-in-6 diluted Sasa veitchii extract, gas sterilized; |
| No.17 | Mixture solution; and |
| No.18 | Untreated. |

Antibacterialness tests were applied to these specimens by means of Staphylococcus aureus (Escherichia coli IFO 3972) to obtain a result as shown in Table 8.

**(Table 8)**

| Specimen (Test Solution) | A | B |
|---|---|---|
| No.15 (1-in-6 diluted Sasa veitchii extract) | 1.10x10⁵ | 9.50x10⁴ |
| No.16 (No.7 Test Solution)(further gas sterilized) | do. | 9.00x10⁴ |
| No.17 (Mixture solution) | do. | <50 |
| No.18 (Untreated) | do. | 1.60x10⁶ |
| (where A and B denote the number of inocula bacteria and the number of bacteria after preservation, respectively.) | | |

From Table 8, it was confirmed that Specimen No.17, containing the mixture solution of the 1-in-10 diluted Sasa veitchii extract and the 1%-butyl-parabenzoic-ester-containing ethanol, exhibited strong antibacterialness against Escherichia coli. However, Specimen No.15, containing the 1-in-6 diluted Sasa veitchii extract, and Specimen No.16, obtained by having Specimen No.15 further gas sterilized, were not that strong, but in a range of slightly stronger than the untreated Specimen 18, in terms of antibacterialness against Escherichia coli.

It is noted that the tests with the 1-in-6 diluted Sasa veitchii extract has revealed a phenomenon that Sasa veitchii extract reacts strongly to strong bacteria, such as Pseudomonas aeruginosa, but reacts weakly to bacteria that are weak and necessary to an extent, such as Escherichia coli. Thus, it can be said that Sasa veitchii extract is best and ideal for use in sticking plaster, and the like.

## Claims

1. A silk cloth for protecting affected parts, comprising a piece of either of knitted-or-woven silk fabric or non-woven silk fabric, made to contain either Sasa veitchii extract or parabenzoic ester, or both thereof.

2. A silk cloth for protecting affected parts, CHARACTERIZED IN: comprising a piece of floss silk (1) that is impregnated with one or more from a group of Sasa veitchii extract, ethanol containing n-butyl-p-hydroxybenzoic acid by 20% or more, and ethanol containing n-propyl-p-hydroxybenzoic acid by 10% or more, and then is dried.

3. A silk cloth for protecting affected parts, CHARACTERIZED IN: comprising a piece of floss silk (1) that is impregnated with either of Sasa veitchii extract or a mixture solution of said Sasa veitchii extract and ethanol containing parabenzoic ester, and then is dried.

4. A silk cloth for protecting affected parts, according to either claim 2 or claim 3, CHARACTERIZED IN THAT: said piece of floss silk (1) is adhered to a piece of non-woven fabric made of either of cellulose, polyester, polyurethane, or the like, or to a piece of non-woven fabric made of cellulose, reinforced with polyurethane or the like.

5. A silk cloth for protecting affected parts, according to claim 1, CHARACTERIZED IN THAT: said parabenzoic ester is contained in ethanol.

6. A silk cloth for protecting affected parts, according to claim 5, CHARACTERIZED IN THAT: said parabenzoic ester is either of n-butyl-p-hydroxybenzoic acid or n-propyl-p-hydroxybenzoic acid, or both thereof.
